# EUROPEAN PATENT APPLICATION

(11) **EP 2 781 194 A1**
(43) Date of publication of application: **24.09.2014**
(21) Application number: 12848931.7
(22) Date of filing: 15.11.2012
(51) Int. Cl.: A61B 17/064, A61B 17/08, A61B 18/12

(54) **SURGICAL INSTRUMENT**

(30) Priority: 15.11.2011 JP 2011249366
(71) Applicant: Suzuki, Naoki, Tokyo 110-0005 (JP)
(72) Inventor: SUZUKI, Naoki, Taito-ku, Tokyo 110-0005 (JP); HATTORI, Asaki, Taito-ku, Tokyo 110-0005 (JP)
(74) Representative: Moore, Graeme Patrick
(86) International application number: PCT/JP2012/079638
(87) International publication number: WO 2013/073609

(57) **Abstract**

Provided is a suture instrument capable of shortening operation time required to ligate a wound, and cauterizing the wound. Provided is a surgical needle including a shape memory alloy which has been shape memory-processed to return, at a temperature equal to or higher than its transformation point, to its original shape selected from a group consisting of a ring shape, a spiral shape, a coil shape and a clinch shape. Use of the surgical needle of the present invention makes it unnecessary to employ a threaded suture needle, shortens the operation time required to ligate an incised part or a wound in an organ such as the digestive tract in the body or the skin or the like, and allows cauterization of the wound.

## Description

### TECHNICAL FIELD

The present invention relates to a surgical instrument for suturing a surgical part such as an incised part or a wound in biological tissue and stopping bleeding from the surgical part, and more particularly to a surgical needle made of a shape memory alloy which has been variously shape memory-processed.

### BACKGROUND ART

Development of various medical instruments is proceeding. For example, a stent constructed of a stainless steel mesh in order to prevent stricture of a blood vessel has been devised, and a stent constructed of a shape memory alloy also has recently been devised. (Refer to Patent Literatures 1 and 2.)

Development of other medical instruments also has advanced, and, for example, various instruments and suture needles have been devised in order to suture a wound in a patient. For example, Patent Literature 3 discloses a suture needle in which a surface of a needle tip has been subjected to blasting in order to reduce piercing resistance when the needle pierces through biological tissue, and Patent Literature 4 discloses a suture needle in which a spiral concave groove is formed in the needle from its distal end side to proximal end side so as to reduce the piercing resistance and thereby provide good suture which is not prone to impose a load on the biological tissue. Also, Patent Literature 5 discloses a surgical needle having a shape memory effect, and Patent Literature 6 discloses a suture thread having the shape memory effect.

However, a conventional surgical needle has required skill and time since the surgical needle, whenever used, has been used in combination with a suture thread to suture an incised part and stop bleeding from the incised part. Further, there has been a demand for quick, safe and reliable immobilization (or cauterization) of biological soft tissue in surgery under a scope or robotic surgery which limits the use of hands.

### CITATION LIST

### PATENT LITERATURE

Patent Literature 1: Japanese Patent Application Publication No. Hei 10-337333
Patent Literature 2: Japanese Patent Application Publication No. 2011-010866
Patent Literature 3: Japanese Patent Application Publication No. 2005-334193
Patent Literature 4: Japanese Patent Application Publication No. 2007-229026
Patent Literature 5: Japanese Patent No. 3700089
Patent Literature 6: Japanese Patent No. 3193971

### SUMMARY OF INVENTION

### TECHNICAL PROBLEM

The present invention has been made in view of the above circumstances. An object of the present invention is to provide a surgical needle capable of shortening operation time by ligating an incised part without the use of a threaded surgical needle, and also capable of cauterizing the incised part.

### SOLUTION TO PROBLEM

As a result of devoting efforts to studies in view of the above object, the inventors have brought the present invention to completion by finding out that, instead of the conventional concept that a thread is used to suture soft tissue, transformation of a shape memory alloy is utilized to suture an incised part or a wound, and also, cauterization of the incised part or the wound is done to immobilize tissue near and around the incised part or the wound.

Specifically, the present invention provides a surgical needle including a shape memory alloy which has been shape memory-processed to return, at a temperature equal to or higher than its transformation point, to its original shape selected from a group consisting of a ring shape, a spiral shape, a coil shape and a clinch shape.

The shape memory alloy is characterized by being shaped in an arcuate shape, a spiral shape or a substantially U-shape. Also, in a preferred embodiment of the present invention, at the temperature equal to or higher than the transformation point, the shape memory alloy returns from the arcuate shape to the ring shape or the spiral shape, or returns from the spiral shape to the coil shape, or is transformed from the substantially U-shape into the clinch shape.

Return of the shape memory alloy to the original shape can be accomplished by heating the shape memory alloy through energization. In the surgical needle of the present invention, suture and/or hemostasis of an incised part can be done by ligation of the incised part or cauterization around the incised part.

Further, desirably, the surgical needle of the present invention is formed in such a manner that its end portion is sharp-edged.

The transformation point of the shape memory alloy is selectable from substantially a range of 30°C to 110°C, depending on a mixing ratio between nickel and metal such as titanium which make up the shape memory alloy. Preferably, the transformation point of the shape memory alloy is set equal to or higher than 40°C, taking suture of biological tissue into account.

### ADVANTAGEOUS EFFECTS OF INVENTION

According to the surgical needle of the present invention, the operation time required to ligate an incised part or a wound in various organs (such as the digestive tract) in the body or the skin or the like can be shortened, and besides, the incised part or the wound can be cauterized, which in turn enables controlling the speed and strength of ligation and a cauterization range. Specifically, the speed of the ligation can be controlled by changing an applied voltage. Also, the strength of the ligation and the cauterization range can be controlled by changing the voltage and energization time.

Use of the surgical needle of the present invention makes it unnecessary to employ a threaded suture needle, shortens the operation time required to ligate an incised part or a wound in an organ such as the digestive tract in the body or the skin or the like, and allows cauterization of the wound.

Also, the surgical needle of the present invention can be used in surgery under a scope such as endoscopic surgery or robotic surgery, thus allowing simple and easy operation of a tool or an instrument (such as a forceps) taken out of a forceps hole in a leading end portion of an endoscope.

### BRIEF DESCRIPTION OF DRAWINGS

[Fig. 1] Fig. 1 is a view of assistance in explaining external appearance shapes of a surgical needle of the present invention.
[Fig. 2] Fig. 2 is a schematic illustration of an embodiment in which the surgical needle of the present invention returns to a ring shape or a spiral shape to suture an incised part.
[Fig. 3] Fig. 3 is a schematic illustration of an embodiment in which the surgical needle of the present invention returns to the ring shape or the spiral shape to suture the incised part.
[Fig. 4] Fig. 4 is a schematic illustration of an embodiment in which the surgical needle of the present invention returns to the ring shape or the spiral shape to suture the incised part.
[Fig. 5] Fig. 5 is a schematic illustration of an embodiment in which the surgical needle of the present invention returns to a clinch shape to suture the incised part.
[Fig. 6] Fig. 6 is a schematic illustration of an embodiment in which the spiral surgical needle of the present invention is used to suture the incised part.
[Fig. 7] Fig. 7 is a schematic illustration of an embodiment in which the surgical needle of the present invention returns to a coil shape to suture the incised part.
[Fig. 8] Fig. 8 is a schematic illustration of an embodiment in which the surgical needle of the present invention returns to the coil shape to suture the incised part.
[Fig. 9] Fig. 9 is a schematic illustration of an embodiment in which the surgical needle of the present invention returns to the coil shape to suture the incised part.
[Fig. 10] Fig. 10 is an illustration of a state in which the surgical needle of the present invention housed in a tube is pushed out of the tube by using a forceps.
[Fig. 11] Fig. 11 is a schematic illustration of an embodiment in which the surgical needle of the present invention pushed out of the tube returns to the coil shape to suture the incised part.

### DESCRIPTION OF EMBODIMENTS

The present invention relates to a surgical needle made of a shape memory alloy which has been shape memory-processed to return to a predetermined shape at a temperature equal to or higher than its transformation point. The surgical needle of the present invention is made of the shape memory alloy, and, after the surgical needle has bitten into soft tissue, the shape memory alloy is energized and thereby heated to return to its original shape, and thereby, a physical ligation effect and a cauterization effect for surrounding soft tissue can be achieved to thus enable closing an incised part and stopping bleeding from the incised part.

The surgical needle which has been shape memory-processed by using the shape memory alloy can be flexibly transformed at a temperature lower than the transformation point (martensite) and returns to the original shape at or above the transformation point (austenite). Thus, for example, if a shape memory alloy having a transformation point of 40°C is shape memory-processed, the shape memory alloy can be flexibly transformed below the transformation point, such as at room temperature, and returns to its original shape when heated to the transformation point or higher.

The surgical needle of the present invention, as compared to a stent for blood vessel, is different from the stent in intended use, approach and mechanism of operation in the following respects. Specifically, the stent for blood vessel assumes a mesh structure, and, after staying in a blood vessel, the stent undergoes a temperature change such as warm water injection thereby to return to its original shape and consequently expand an inner diameter of the blood vessel.

Meanwhile, the surgical needle of the present invention is characterized in that (i) its use is intended for ligation and hemostasis, (ii) the approach involves inserting the needle into a soft part such as biological tissue, and thereafter heating a suture instrument by energizing the suture instrument through a forceps, tweezers or the like, and (iii) the mechanism of operation involves using a shape restoring force of the shape memory alloy to ligate an incised part or a wound, and utilizing the effect of cauterizing the surroundings of the incised part or the wound.

Further, the surgical needle of the present invention is configured so that the needle in itself bites into soft tissue and returns to the original shape to suture an incised part, and the surgical needle of the present invention is different from the conventional surgical needle and suture thread (refer to Patent Literatures 5 and 6, for example) in that the surgical needle of the present invention does not use a suture thread in itself.

The shape of the surgical needle of the present invention is not particularly limited, provided only that it permits suture of biological tissue, and the shape memory alloy which has been shape memory-processed is shaped in such a shape as to facilitate suture of an incised part and a cauterization operation for surrounding tissue. For example, preferably, the shape after returning to the original shape is a ring shape, a spiral shape, a coil shape or a clinch shape.

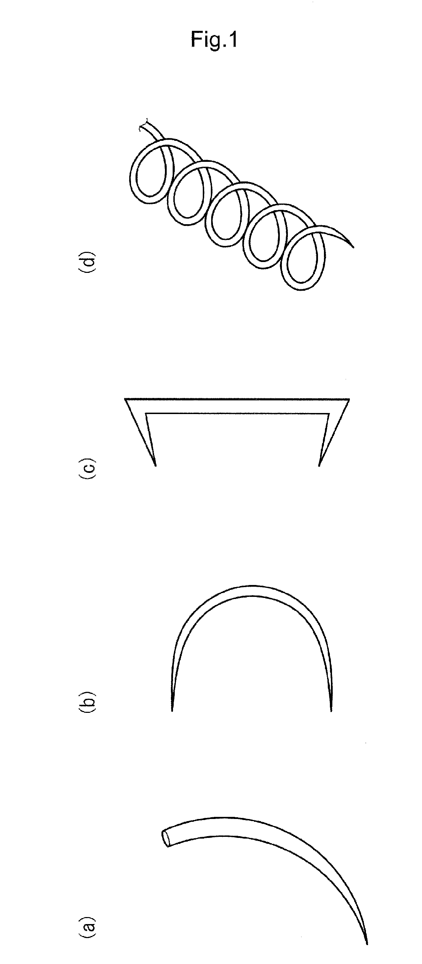

Also, the shapes before returning to the original shape include a sharply or gently curved sharp (or an arcuate shape) given to a surgical sharp or round needle or the like, the shape (or a substantially C-shape) of an arcuate needle having sharp-edged ends, a spiral shape, and a substantially U-shape, in order that the needle can easily bite into soft tissue in the incised part. (See Fig. 1.)

Return of the shape memory-processed surgical needle to the original shape can be accomplished by heating the shape memory alloy to its transformation point or higher. There are various methods for heating the shape memory alloy to the transformation point or higher; for example, the surgical needle may be energized through tweezers or a forceps capable of power supply thereby to produce Joule heat and utilize the Joule heat to heat the shape memory alloy to the transformation point or higher. An electrode may be connected beforehand to the tweezers or the forceps so as to enable supplying a current. Also, after the insertion of the surgical needle of the present invention into biological tissue around an incised part or a wound, an electrode terminal capable of current supply may be connected to the surgical needle to apply the current to the surgical needle. Incidentally, heat may also be supplied to the surgical needle to increase the temperature of the surgical needle to the transformation point or higher and allow the surgical needle to return to the original shape.

Description will be given below with regard to more specific embodiments of the present invention.

Fig. 1 is an external view illustrating external appearance of the surgical needle of the present invention shaped in various shapes. As illustrated in Fig. 1, the surgical needle of the present invention is configured so that the shape memory alloy which has been shape memory-processed in a predetermined shape can be shaped for example in an arcuate shape (see Fig. 1(a) and (b)) such as a typical shape of surgical needle or a crescent shape (or a substantially C-shape), a substantially U-shape (see Fig. 1(c)), or a spiral shape (see Fig. 1(d)). The shape memory-processed alloy can be shaped in any of the shapes illustrated in Fig. 1; however, preferably, the shape memory alloy which has been shape memory-processed in the ring shape or the spiral shape is shaped in the arcuate shape, the shape memory alloy which has been shape memory-processed in the clinch shape is shaped in the substantially U-shape, and the shape memory alloy which has been shape memory-processed in the coil shape is shaped in the spiral shape, taking into account the effect of ligating an incised part, or the like.

The shape memory alloy has the property of returning to its original shape when the shape memory alloy reaches a temperature equal to or higher than its transformation point, and the surgical needle of the present invention can utilize the property to ligate an incised part or a wound. Further, the shape memory alloy is of relatively high electrical resistance and thus produces Joule heat during energization. The shape of the shape memory alloy can return from martensite to austenite by the heat produced by the energization.

The shape memory alloy is an alloy of nickel and metal selected from titanium, aluminum, cobalt, iron, manganese and the like, and varies in molecular structure from one to another of two phases, namely, the martensite and the austenite. For example, a typical shape memory alloy assumes the martensite below 25°C and assumes the austenite at or above 32°C; however, this can be adjusted by changing a mixing ratio between nickel and titanium or the like and may be changed as appropriate for adaptation to the required transformation point. To shape memory-process the shape memory alloy, a wire of the shape memory alloy is wound around a jig having a predetermined shape or the alloy is charged into a mold, and the whole jig or mold is put in an oven and is subjected to heat treatment (at about 400°C to 500°C for about an hour). After the heat treatment, the shape memory alloy is removed from the jig or the mold, and the shape memory alloy can be shaped in the desired one of the shapes illustrated in Fig. 1.

Preferably, the surgical needle of the present invention has the transformation point in a range of 40°C to 100°C. When the surgical needle has such a transformation point, the surgical needle is transformed from the martensite into the austenite to return to the original shape, for example by heating the surgical needle to at least 40°C or higher. Also, the proportion of nickel in the shape memory alloy may be reduced to increase the transformation point to about 50°C, thereby preventing the surgical needle from returning to the original shape after merely suturing biological tissue in a patient, and also achieving a structure such that the surgical needle does not return to the original shape unless the surgical needle is externally heated to 50°C or higher. The surgical needle which achieves greater ease of use can be provided by setting the transformation point of the shape memory alloy in this manner. The transformed surgical needle stays in an incised part or a wound to thus close the incised part or the wound and hence enable preventing tissue therearound from becoming damaged.

Heating temperature of the shape memory alloy can be controlled by changing electric power applied to a resistor, since energization is based on the electric power. Specifically, the amount of Joule heat is proportional to the product of resistance R and the square of current I, and thus, the amount of heat produced can be adjusted by utilizing this relationship. A temperature for cauterization of soft tissue around an incised part lies for example between 50°C and 100°C inclusive, or is preferably equal to or higher than 70°C.

Next, the shape memory-processed surgical needles will be described more specifically.

Each of the surgical needles having the shapes illustrated in Fig.1 (a) and (b) is shape memory-processed in such a manner that the curvature of an arc is large or small, and thereby, the depth of suture of an incised part or a wound can be adjusted. For example, when a person who performs surgery uses the tweezers (or the forceps) or the like to suture the wound and the person wants to insert the needle into tissue to a great depth, the person can cause the leading end of the needle to reach deep into the tissue to suture the wound, by using the surgical needle processed in such a manner that the curvature is relatively small. Also, when the person wants to insert the needle into the tissue to a shallow depth, the person can suture the wound in the tissue at the shallow depth by using the surgical needle processed in such a manner that the curvature is relatively large.

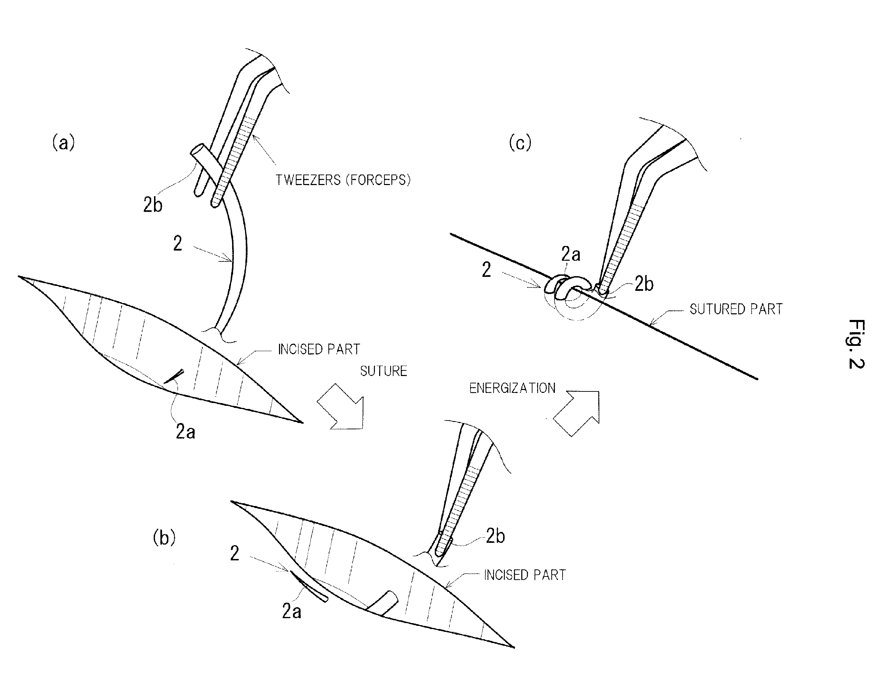

Fig. 2 is an illustration of assistance in explaining how the surgical needle illustrated in Fig. 1(a) is used to treat an incised part. As illustrated in Fig. 2 (a), a surgical needle 2 whose temperature does not reach its transformation point, before returning to its original shape, has the arcuate shape (or the shape of surgical needle in common use), and the surgical needle 2 is formed in such a manner that its leading end 2a is sharp-edged. The surgical needle 2 is gripped for example by the tweezers or the forceps or the like, and the leading end 2a is inserted into soft tissue around an incised part or a wound. The leading end 2a of the surgical needle 2 inserted from one side of the wound is further inserted toward the other side of the incised part, and thereby, the leading end 2a can appear from tissue on the other side (see Fig. 2(b)). After the surgical needle 2 has pierced through the incised part, the surgical needle 2 is energized to produce heat. Then, when the temperature rises to the transformation point or higher, the surgical needle 2 returns to the original shape at the time of shape memory processing (see Fig. 2(c)). Fig. 2(c) illustrates a state in which the surgical needle 2 returns to the spiral shape in the form of approximately two turns thereby to suture the incised part. The surgical needle 2 may also be shape memory-processed to return from the arcuate shape to the ring shape (less than two turns) or the spiral shape (two turns or more) according to the size of the incised part or the like. When returning to the original shape, the surgical needle 2 closes the incised part, and the surgical needle 2 is continuously energized with predetermined power thereby to cauterize and immobilize the soft tissue around the incised part.

Incidentally, besides the embodiment of the shape memory processing and shaping illustrated in Fig. 2, the surgical needle which has been shape memory-processed in the clinch shape may be shaped in the arcuate shape for use.

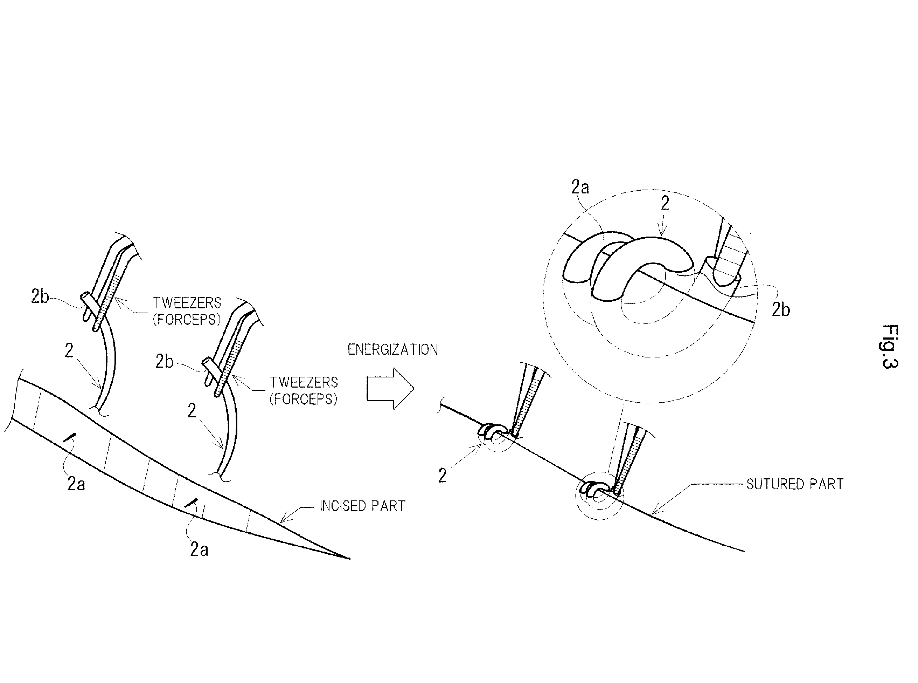

Fig. 3 is an illustration of an embodiment in which the plural surgical needles illustrated in Fig. 2 are used to treat an incised part. Fig. 3 illustrates a case where the two surgical needles 2 are used.

As illustrated in Fig. 3, the plural surgical needles 2 may be used according to the size of a wound. For example, the one surgical needle 2 is used when an incised part is so small as to be closed with a suture; meanwhile, the plural surgical needles 2 are used for a long thin and large incised part according to the size of the incised part, and thereby, the incised part can be closed with higher reliability. Incidentally, plural surgical needles having other shapes may also be used in the same manner.

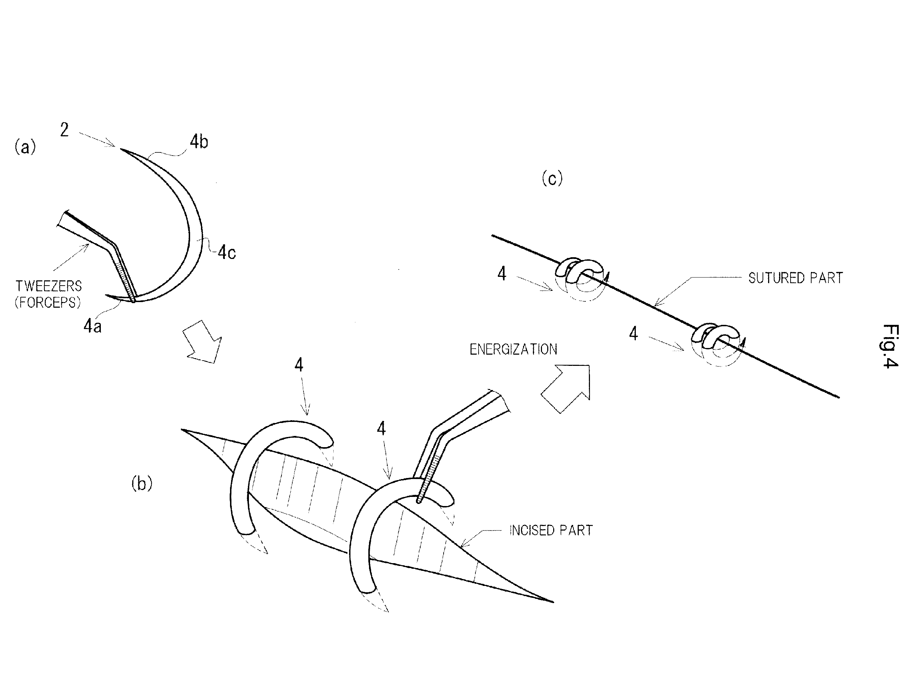

Fig. 4 illustrates an embodiment in which the shape memory alloy is shape memory-processed to return to the spiral shape, and an incised part is sutured and cauterized by using the surgical needle shaped in the shape illustrated in Fig. 1(b) (or an arcuate surgical needle having sharp-edged ends). As illustrated in Fig. 4(a), a surgical needle 4 has sharp-edged ends 4a, 4b and has an arcuate shape in which the ends 4a, 4b are separated from each other at a temperature lower than the transformation point, and thus, the surgical needle 4 is configured to easily pierce through tissue. Although the curvature radius of an arc is not particularly limited, surgical needles having various curvature radii may be prepared for adaptation to various sizes of incised parts or wounds. Also, the shape after returning to the original shape may be the ring shape, the spiral shape or the clinch shape in the same manner as above described, and may be selected as appropriate according to what purpose the surgical needle is used for.

As illustrated in Fig. 4(b), the ends 4a, 4b are pressed against soft tissue around the incised part so that the leading end of the needle bites into the soft tissue, and thereafter, the surgical needle 4 is energized through the tweezers or the like thereby to return to the original shape (see Fig. 4(c)). At this time, as the surgical needle 4 returns to the spiral shape (as illustrated as the form of two turns in Fig. 4(c)), the incised part is closed. The surgical needle 4 is further energized thereby to cauterize and immobilize the soft tissue around the incised part.

Here, Fig. 4 illustrates the embodiment in which the ends of the needle bite into the soft tissue and the needle returns to the original shape; however, operation may be performed so that only one end of the needle bites into the soft tissue, and the other end of the needle bites into the soft tissue by a restoring force produced during transformation by energization. For example, only one end portion of the surgical needle 4 pierces through the soft tissue, and then, in this state, the surgical needle 4 is energized through the tweezers or the forceps thereby to return to the spiral shape in the form of two turns. During two turns of the needle, the other end portion of the surgical needle 4 can pierce through the soft tissue to suture a wound in conjunction with the one end portion of the surgical needle 4 which has previously pierced through the biological tissue.

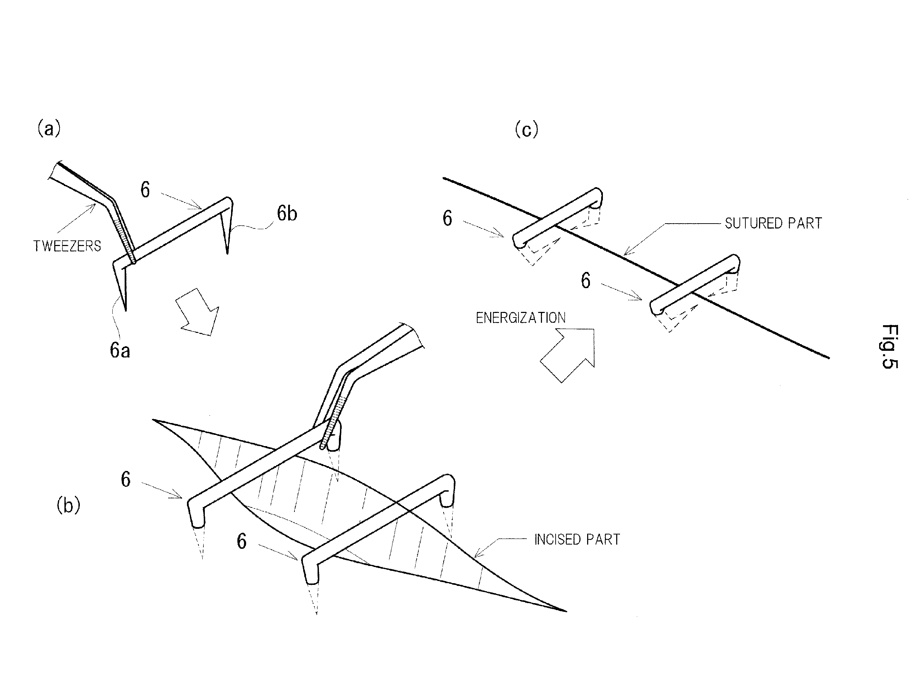

Fig. 5 illustrates an embodiment in which the shape memory alloy is shape memory-processed to return to the clinch shape, and an incised part is sutured and cauterized by using the surgical needle shaped in the substantially U-shape illustrated in Fig. 1 (c). As employed herein, the "clinch shape" refers to a shape when the leading ends of the needle bent in the substantially U-shape are bent facing each other, and the "clinch shape" is a shape like a staple of a stapler when stapling. In this embodiment, the shape memory alloy is shaped in the substantially U-shape to form a surgical needle 6, and the surgical needle 6 is formed in such a manner that its ends 6a, 6b are sharp-edged (see Fig. 5 (a)). When the ends 6a, 6b of the surgical needle 6 pierce through both sides of the incised part (see Fig. 5(b)) and the surgical needle 6 is energized to return to the original shape, the leading ends of the needle are bent facing each other inwardly, thereby to suture the incised part and further cauterize the incised part (see Fig. 5(c)). Also, the shape after returning to the original shape may be the ring shape or the spiral shape besides the clinch shape in the same manner as above described, and may be selected as appropriate according to what purpose the surgical needle is used for.

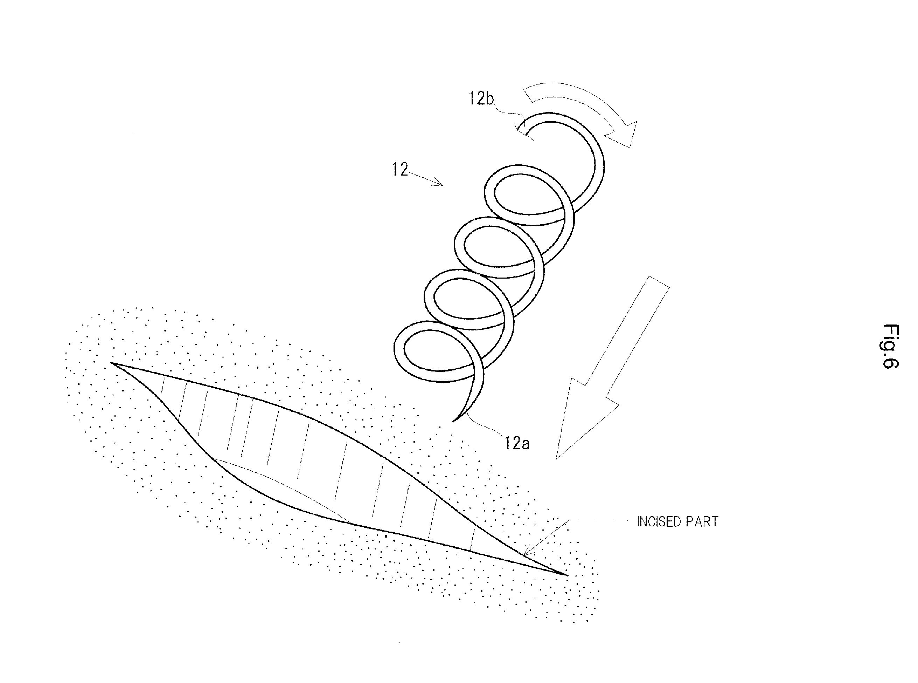

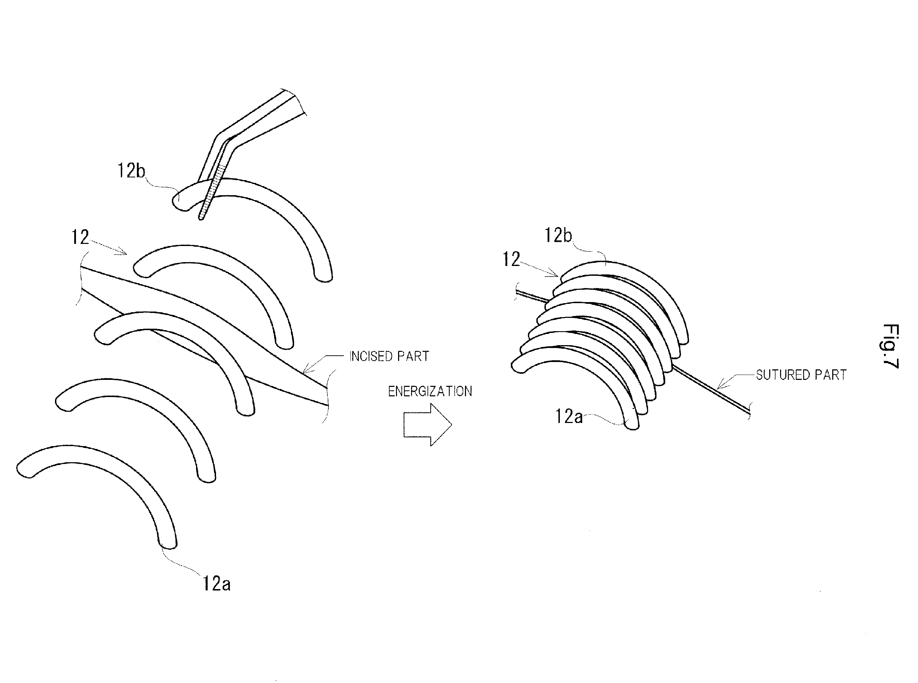

Fig. 6 illustrates an embodiment in which the shape memory alloy is shape memory-processed to return to the coil shape, and an incised part is sutured and cauterized by using the surgical needle shaped in the spiral shape illustrated in Fig. 1(d), and Fig. 7 illustrates an embodiment in which the shape memory alloy is shape memory-processed to return to the coil shape, and an incised part is sutured and cauterized by using the surgical needle shaped in the spiral shape illustrated in Fig. 1 (d).

As illustrated in Fig. 6, a surgical needle 12 (or a coil-shaped surgical needle) which has been shape memory-processed in the coil shape has the spiral shape below its transformation point, and the surgical needle 12 is formed in such a manner that its leading end 12a is sharp-edged. The inner diameter and spiral pitch (or coil pitch) of the surgical needle 12 are not particularly limited, and various pitches or curvature radii may be set according to the size or opening of the incised part or wound.

The surgical needle 12 may pierce through the incised part while rotating from one side thereof toward the other side thereof, or may suture the wound while rotating along the incised part. Details will be described later.

In the embodiments illustrated in Figs. 6 and 7, here, the "coil shape" and the "spiral shape" are terms which relatively express a difference between the shapes of the shape memory alloy before and after returning to the original shape; herein, the shape having a wide spiral pitch, before returning to the original shape, is called the "spiral shape," and the shape in which the spiral pitch is narrower than that before returning to the original shape, after returning to the original shape, is called the "coil shape." For example, in Fig. 7, the surgical needle 12 on the left-hand side of the drawing is in a state before energization (or before returning to the original shape), and its shape is the "spiral shape." On the other hand, the surgical needle 12 on the right-hand side of the drawing is transformed by the energization in such a manner that the spiral pitch becomes narrower, and its shape is the "coil shape."

As illustrated in Fig. 7, the surgical needle 12 illustrated in Fig. 6 can pierce through the incised part from one side thereof to the other side thereof, for example by rotating from the one side toward the other side. In this case, the surgical needle 12 may be partially exposed to the outside to enable connection to the tweezers or the forceps.

After the entry of the leading end 12a of the surgical needle 12 into the other side of the incised part, a current is passed through the surgical needle 12 via the forceps or the tweezers. When energization starts, heat is produced according to metal resistance of the shape memory alloy which forms the surgical needle 12. When the temperature of the surgical needle 12 rises to the transformation point or higher by the produced heat, the spiral pitch becomes narrower, and the surgical needle 12 returns from the spiral shape to the coil shape. In addition, the wound can be cauterized by further energization.

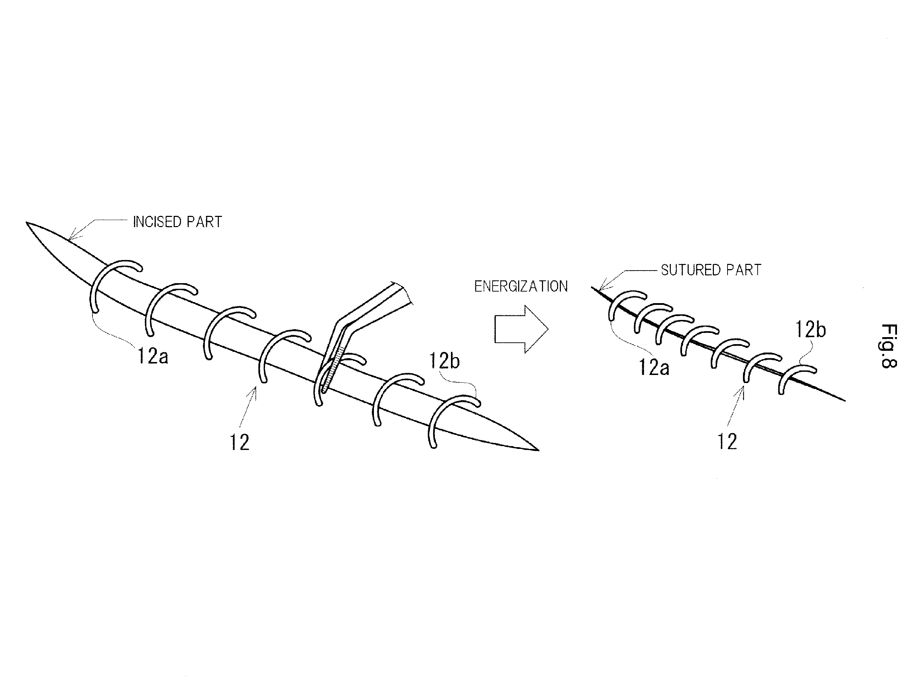

In Figs. 6 and 7, the surgical needle 12 pierces through the incised part from the one side thereof to the other side thereof; however, how the surgical needle 12 pierces through the incised part is not limited to the above. For example, as illustrated in Fig. 8, the surgical needle 12 may pierce (or be inserted) through a long thin incised part from one end thereof toward the other end thereof. In this manner, the surgical needle 12 is inserted through the long thin incised part from the one end side thereof toward the other end side thereof, and thereby, although the single surgical needle 12 is used, the incised part can be sutured at plural locations, and even the long thin incised part can be sutured and cauterize with reliability.

In the present invention, the shape memory alloy is shape memory-processed in such a manner that the spiral pitch becomes narrower, and the shape memory alloy may be shape memory-processed in such a manner that the curvature radius of the spiral becomes smaller (see Fig. 8), and thereby, the incised part can be sutured with higher reliability.

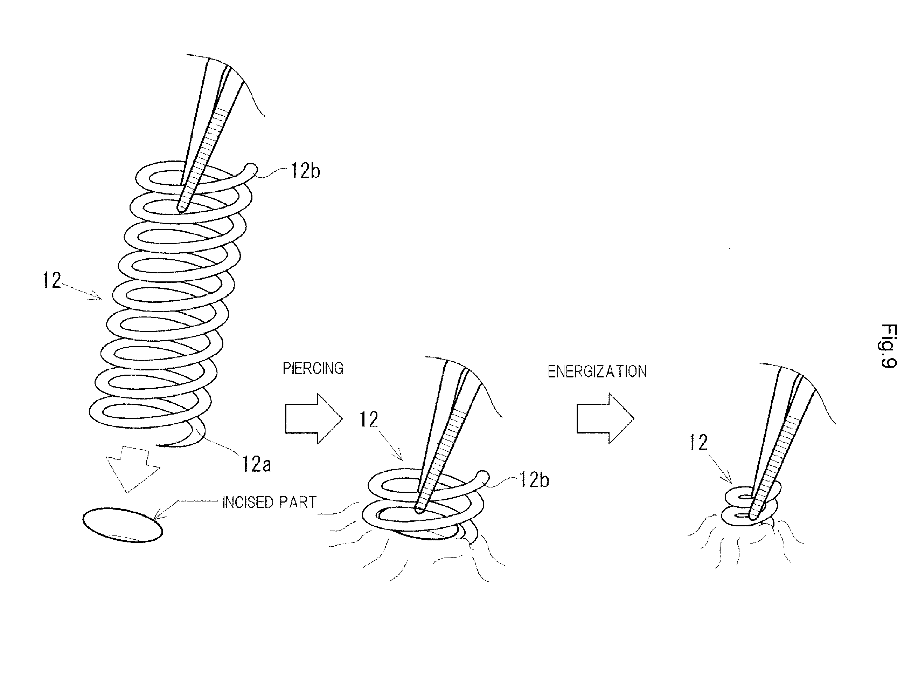

Also, the surgical needle 12 of the present invention may be used for a spot-shaped incised part or wound, as illustrated in Fig. 9. Fig. 9 illustrates an embodiment in which the surgical needle 12 is used and is screwed into the spot-shaped incised part from its shallow portion to deep portion. The surgical needle 12 is inserted from above into the periphery of the spot-opening incised part while rotating, and, after the insertion of the surgical needle 12 into the periphery of the incised part, the surgical needle 12 is energized thereby to allow closing the incised part. The incised part can also be cauterized by further energization, and thereby, bleeding from the spot-shaped wound can be stopped.

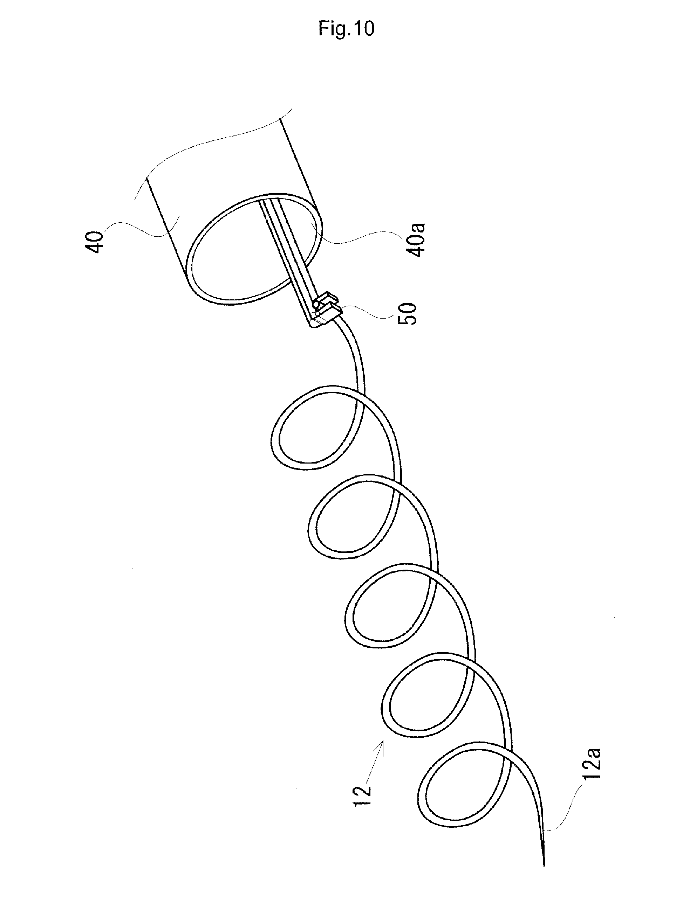

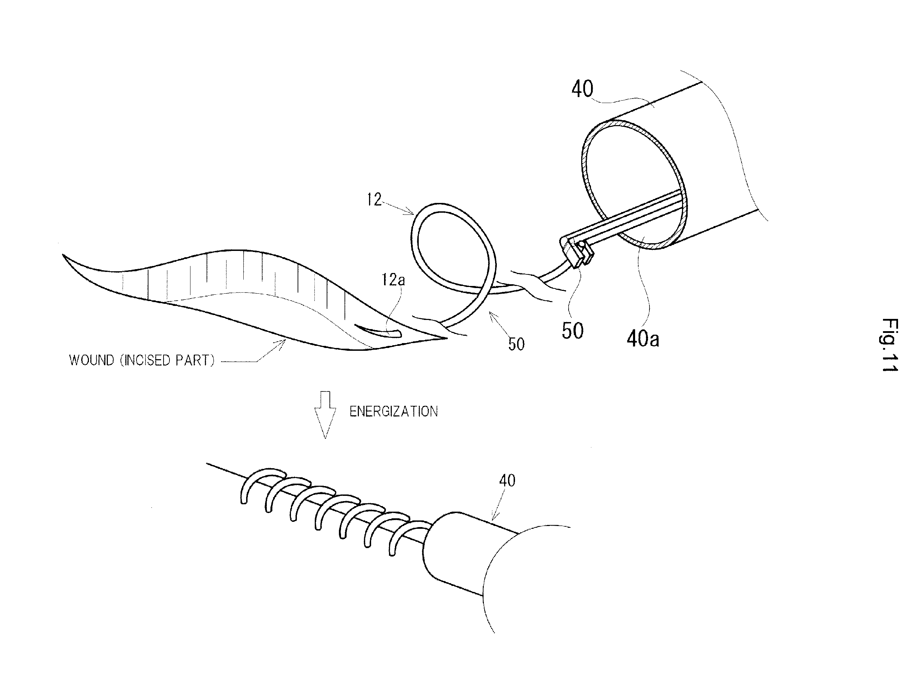

Next, description will be given with reference to Figs. 10 and 11 with regard to an embodiment in which the surgical needle of the present invention formed in the spiral shape is used in surgery under a scope. Fig. 10 illustrates a state in which the spiral surgical needle housed in a tube such as an endoscope is pushed out by using the forceps. Also, Fig. 11 illustrates a state in which the leading end of the surgical needle pushed out of the tube is inserted into the periphery of an incised part by the forceps thereby to treat the incised part.

As illustrated in Fig. 10, a tube 40 which is inserted into the body in surgery under a scope such as endoscopic surgery can contain the spiral surgical needle 12. A forceps 50 capable of gripping and releasing the surgical needle 12 is movably provided in the tube 40, and the surgical needle 12 can be operated by the forceps 50. To suture an incised part in internal organ tissue in the endoscopic surgery or the like, the tube 40 is inserted into the body from outside, and the surgical needle 12 gripped by the forceps 50 is fed out of an opening 40a in the leading end of the tube 40. As illustrated in Fig. 11, the leading end 12a of the surgical needle 12 gripped by the forceps 50 pierces through tissue around the incised part. For example, the surgical needle 12 piercing through the tissue is rotated in such a manner that the leading end 12a alternately pierces through the incised part on both sides thereof, and thereby, the incised part can be sutured at plural locations along the incised part, as illustrated in Fig. 8. The surgical needle 12 which has sutured the incised part is energized through the forceps 50, and thereby, the spiral surgical needle 12 is transformed for example into a reduced-diameter and/or coil shape to close the incised part. The surgical needle 12 is further energized thereby to cauterize the tissue around the incised part and stop bleeding from the incised part.

As described above, the surgical needle of the present invention can be used in endoscopic surgery or robotic surgery as well as manual operation. There are various instruments for the endoscopic surgery and various devices for use in the robotic surgery, and, for example, when ligating an incised part or a wound, a person who performs surgery is required to have highly technical skill; however, the use of the surgical needle of the present invention enables achieving easy and quick ligation of the incised part or the wound. Further, according to the surgical needle of the present invention, not only ligation but also cauterization can be simultaneously performed. For example, an operation for ligating the incised part has heretofore been complicated; specifically, one end of a thread is held by one of the left and right forceps so as to form a ring, and the other end of the thread is passed through the ring by the other forceps. However, the use of the suture instrument of the present invention enables easily closing the incised part.

Also, the surgical needle of the present invention is of a stay type, does not need a thread for suture, eliminates a need to thread a suture needle, involved in the conventional suture needle, and enables providing a medical suture instrument which achieves greater ease of use. Also, suture and cauterization of a wound can be simultaneously performed, and a treatment effect which is not to be found in the prior art can be expected.

### REFERENCE SIGNS LIST

- 2: surgical needle
- 2a: leading end
- 2b: rear end
- 4: surgical needle
- 6: surgical needle
- 12: coil-shaped surgical needle
- 12a: leading end
- 12b: rear end
- 40: tube
- 40a: opening
- 50: forceps

## Claims

1. A surgical needle, comprising a shape memory alloy which has been shape memory-processed to return, at a temperature equal to or higher than its transformation point, to its original shape selected from a group consisting of a ring shape, a spiral shape, a coil shape and a clinch shape.

2. The surgical needle according to claim 1, wherein the shape memory alloy is shaped in an arcuate shape, a spiral shape or a substantially U-shape.

3. The surgical needle according to claim 1, wherein the shape memory alloy is energized and thereby heated to return to the original shape.

4. The surgical needle according to claim 1, wherein the shape memory alloy returns from the arcuate shape to the ring shape or the spiral shape at the temperature equal to or higher than the transformation point.

5. The surgical needle according to claim 1, wherein the shape memory alloy returns from the spiral shape to the coil shape at the temperature equal to or higher than the transformation point.

6. The surgical needle according to claim 1, wherein the shape memory alloy is transformed from the substantially U-shape into the clinch shape at the temperature equal to or higher than the transformation point.

7. The surgical needle according to any one of claims 1 to 6, wherein suture and/or hemostasis of an incised part are done by ligation of the incised part or cauterization around the incised part.
